Europäisches Patentamt

European Patent Office

Office européen des brevets

(19)

(11) Numéro de publication : **0 370 909 B1**

# (12) FASCICULE DE BREVET EUROPEEN

(45) Date de publication du fascicule du brevet :
**10.03.93 Bulletin 93/10**

(51) Int. Cl.$^5$ : **C07C 11/00, C07C 2/76**

(21) Numéro de dépôt : **89403234.1**

(22) Date de dépôt : **22.11.89**

(54) **Procédé de conversion de gaz naturel ou d'alcanes légers en hydrocarbures insaturés.**

(30) Priorité : **24.11.88 FR 8815362**

(43) Date de publication de la demande :
**30.05.90 Bulletin 90/22**

(45) Mention de la délivrance du brevet :
**10.03.93 Bulletin 93/10**

(84) Etats contractants désignés :
**AT BE CH DE ES GB GR IT LI LU NL SE**

(56) Documents cités :
**FR-A- 1 323 474**
**FR-A- 2 596 046**

(73) Titulaire : **GAZ DE FRANCE**
**23, rue Philibert Delorme**
**F-75017 Paris (FR)**

(72) Inventeur : **Nikravech, Mehrdad**
**285 rue Saint-Jacques**
**F-75005 Paris (FR)**
Inventeur : **Vedrenne, Isabelle, Jeanine**
**32 Quai de la Loire**
**F-75019 Paris (FR)**
Inventeur : **Amouroux, Jacques**
**13bis rue de Gilleziers**
**F-91440 Bures sur Yvette (FR)**
Inventeur : **Saint-Just, Jacques Jean**
**40bis rue du Président Wilson**
**F-78230 Le Pecq (FR)**

(74) Mandataire : **Durand, Yves Armand Louis et al**
**CABINET WEINSTEIN 20, Avenue de**
**Friedland**
**F-75008 Paris (FR)**

EP 0 370 909 B1

## Description

La présente invention se rapporte à un procédé de conversion de gaz naturel ou d'alcane (s) léger (s) en hydrocarbures insaturés et plus particulièrement à un procédé de conversion avec apport d'énergie électrique appliqué notamment dans les industries chimiques et énergétiques.

Il existe actuellement des procédés de conversion avec apport d'énergie électrique. Un procédé représentatif est le procédé Hüls qui permet la conversion d'hydrocarbures tels que le méthane en hydrocarbures insaturés et notamment en acétylène. Ce procédé consiste à faire passer du méthane dans un arc électrique puis à séparer les produits obtenus. Cependant ce procédé a pour inconvénient de former une quantité très importante de noir de carbone.

Aussi, une amélioration a été proposée en utilisant un plasma d'hydrogène. Le plasma d'hydrogène permet d'apporter l'énergie nécessaire à la réaction de conversion du méthane, qui est une réaction endothermique. Cette énergie est apportée in situ par les gaz plasmagènes sans l'intermédiaire d'une paroi. Mais si le plasma est une source d'énergie dans la réaction de conversion du méthane il présente encore des inconvénients car sa température est trop importante pour la réaction envisagée. En effet, le méthane porté à une température supérieure à 1200°C se décompose par une succession de réaction de déshydrogénations et de cyclisations en un mélange de substances polyaromatiques conduisant au noir de carbone.

Ces procédés ne sont donc pas complètement satisfaisants car ils impliquent une formation trop importante de noir de carbone de qualité non contrôlée qui devient donc un sous-produit difficilement valorisable.

L'invention a pour but un procédé qui ne présente pas les difficultés et les inconvénients des procédés connus.

Pour atteindre ce but, l'invention est caractérisée en ce que l'on créé à l'intérieur d'un espace de réaction un lit fluidisé de particules d'un matériau réfractaire et avantageusement catalytique et en ce que l'on introduit un plasma d'un gaz contenant de l'hydrogène et le gaz naturel ou le ou les alcanes légers dans le lit de façon que celui-ci effectue une trempe du milieu réactionnel et catalyse la réaction de conversion.

Selon une caractéristique de l'invention, le lit de particules est fluidisé par un courant gazeux de fluidisation contenant avantageusement de l'hydrogène.

Selon encore une autre caractéristique de l'invention, le courant gazeux de fluidisation contient de l'hydrogène et de l'argon.

Selon un mode de réalisation préférentiel de l'invention, le gaz naturel ou le ou les alcanes légers est introduit dans le lit avec le courant gazeux de fluidisation et l'alcane léger est le méthane. L'hydrogène de fluidisation et le méthane sont introduits dans le lit en une proportion hydrogène/méthane allant de 0,5 à 10 et de préférence de 2 à 5.

Selon une autre caractéristique de l'invention, le courant gazeux de fluidisation est préchauffé en amont du lit à une température comprise entre 50°C et 500°C et de préférence entre 150°C et 350°C.

Selon une particularité du procédé de l'invention, on introduit un plasma contenant au moins 10% d'hydrogène et pouvant contenir de l'argon.

Selon une autre particularité de l'invention, le lit est constitué de particules d'un matériau choisi notamment dans le groupe consistant en oxydes, carbures, nitrures et borures.

Selon une autre particularité de l'invention, les particules produisent un effet catalytique.

Selon encore une autre particularité de l'invention, le lit contient de plus un catalyseur.

La réaction de conversion est réalisée dans le lit à une température comprise entre 500°C et 1200°C et de préférence entre 500°C et 800°C.

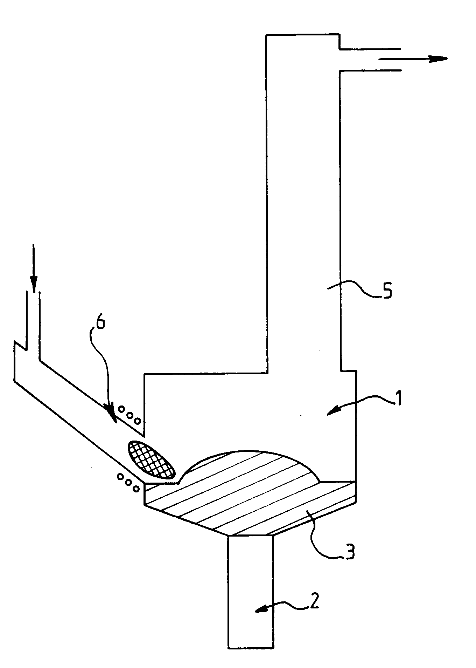

L'invention sera mieux comprise et d'autres buts, caractéristiques, détails et avantages de celle-ci apparaîtront plus clairement au cours de la description explicative qui va suivre faite en référence à la figure unique représentant un schéma d'un mode de réalisation préférentiel de l'invention.

Le procédé de l'invention est mis en oeuvre à l'aide d'un dispositif du type de celui représenté à la figure annexée et comprenant une enceinte 1 comportant au niveau de son fond des moyens d'injection 2 d'un courant gazeux de fluidisation, des moyens de sortie de ce dernier (non représentés) et contenant une masse de particules d'un matériau destinées à former un lit fluidisé 3, et une torche à plasma 6 d'un gaz contenant de l'hydrogène, adaptée pour introduire le plasma à l'intérieur de l'enceinte dans le lit de particules fluidisé.

Il est prévu un tube d'évacuation 5 relié à la sortie de l'enceinte.

La torche à plasma est raccordée au niveau d'une paroi de l'enceinte de façon que le plasma soit introduit dans le lit fluidisé. On peut faire varier l'angle d'introduction de la torche dans l'enceinte de 0° à 90°. De préférence, l'angle d'introduction de la torche est de 20° par rapport à la section horizontale de l'enceinte. Typiquement cette torche est constituée de deux tubes concentriques en silice, d'un diamètre extérieur de 30 mm, entourés de cinq spires inductives creuses en cuivre refroidies à l'eau, parcourues par un courant électrique de fréquence élevée.

La paroi interne de l'enceinte 1 est par exemple en alumine réfractaire de 4 mm d'épaisseur, calorifugée par une couche de 20 mm de briques poreuses. L'ensemble est recouvert de laine de verre et d'un ruban d'amiante. Les dimensions de l'enceinte plus importantes que celles du plasma évitent le contact direct des parois avec la zone chaude du plasma. L'enceinte comporte une zone pyramidale dans laquelle les particules sont mises en suspension et des thermocouples (non représentés) sont installés dans l'enceinte pour mesurer la température du lit fluidisé. Un rodage hémisphérique (non représenté) installé sur l'un des côtés de l'enceinte, permet l'introduction du plasma traitant ainsi l'ensemble des particules.

Les moyens d'injection 2 du courant gazeux de fluidisation comprennent par exemple un tube de silice opaque de 40 mm entouré d'un ruban chauffant et rempli de billes réfractaires, ce sytème permet le préchauffage des gaz et un thermocouple (non représenté) est prévu dans le tube pour contrôler la température des gaz de fluidisation.

Le tube d'évacuation 5 est par exemple constitué par un tube de quartz de 85 mm de diamètre et de 500 mm de longueur et des thermocouples (non représentés) sont installés dans ce tube pour mesurer la température de courant gazeux le traversant. La sortie de ce tube peut être reliée à un échangeur thermique à eau (non représenté) dans lequel le mélange réactionnel est refroidi avant d'être prélevé pour analyse.

Le lit est constitué de particules d'un matériau choisi notamment dans le groupe consistant en oxydes, carbures, nitrures, et borures. On peut en dresser à titre d'exemples la liste suivante :

| | |
|---|---|
| - oxydes d'aluminium | $Al_2O_3$ |
| de magnésium | $MgO$ |
| de calcium | $CaO$ |
| de béryllium | $BeO$ |
| de cérium | $CeO$ |
| de thorium | $ThO_2$ |
| d'hafnium | $HfO_2$ |
| de lanthane | $La_2O_3$ |
| et autres oxydes mixtes | |
| - carbures de silicium | $SiC$ |
| de thorium | $ThC$ |
| de bore | $B_4C$ |
| - nitrures de bore | $BN$ |
| d'hafniumm | $HfN$ |
| de zirconium | $ZrN$ |
| - borures de thorium | $ThB_4$ |
| de niobium | $NbB_2$ |
| de zirconium | $ZrB_2$ |

- carbone (graphite) C

Quelle que soit la nature des matériaux utilisés, ceux-ci doivent être réfractaires car les particules du lit doivent pouvoir résister à des températures élevées puisqu'elles sont en contact avec le jet de plasma. Les particules du lit peuvent elles même jouer le rôle de catalyseur et il est également possible de leur adjoindre un autre catalyseur.

Il faut bien comprendre que le mot "catalyseur" est pris dans son sens large c'est-à-dire que les particules peuvent accélérer certaines réactions souhaitées ou inhiber certaines réactions non souhaitées comme la formation de noir de carbone ou coke.

Dans le procédé de la présente invention, les particules du lit sont mises en fluidisation en un lit jaillissant par le débit d'un courant gazeux de fluidisation formé principalement d'hydrogène ou d'un mélange d'hydrogène et d'argon introduit dans l'enceinte 1 à l'aide des moyens d'injection 2 et on introduit dans le lit ainsi fluidisé le gaz naturel ou le ou les alcanes légers devant être convertis. De préférence, comme représenté dans le dispositif de la figure, le gaz naturel ou le ou les alcanes légers est introduit dans le lit fluidisé avec le courant gazeux de fluidisation. On détermine la quantité optimale d'hydrogène de fluidisation de façon à minimiser la formation de noir de carbone. Les gaz de fluidisation sont préchauffés en amont du lit, dans le tube 2, à une température comprise entre 50°C et 500°C et de préférence entre 150°C et 350°C.

La torche à plasma 6 injecte un plasma d'hydrogène, pouvant contenir de l'argon et contenant au moins 10% d'hydrogène, dans le lit de particules fluidisé où s'effectue un transfert homogène de la chaleur entre le plasma et lit fluidisé, permettant ainsi la réalisation d'une réaction de conversion en présence d'hydrogène radicalaire à une température ajustée qui demeure notablement inférieure à celle du plasma, ce qui minimise donc la formation de noir de carbone. Les hydrocarbures insaturés obtenus par la réaction de conversion réalisée à l'intérieur du lit fluidisé sont ensuite évacués par le tube 5. Des thermocouples installés dans ce tube permettent de mesurer les températures.

L'utilisation d'un lit fluidisé dans le procédé selon l'invention présente des avantages importants pour les raisons suivantes :

- ses propriétés de transfert de chaleur permettent une trempe efficace du plasma ;
- sa viscosité sensiblement égale à celle du plasma assure un très bon mélange entre celui-ci et le lit fluidisé ; et
- ses propriétés catalytiques éventuelles peuvent assurer la transformation directe du ou des produits à convertir en hydrocarbures insaturés.

D'autre part, la nature du matériau des particules constituant le lit et/ou la nature du catalyseur permettant d'orienter la conversion vers les produits souhaités.

Les huit exemples suivants sont donnés dans le but de bien mettre en évidence les avantages de la présente invention.

D'une manière générale, les exemples ont été réalisés de la façon suivante :

La torche à plasma fonctionne à une fréquence de 5 MHz pour une puissance de 4,4 kW. L'angle d'injection est de 20°. Les gaz plasmagènes introduits sont de l'argon, à un débit de 30 l/min et de l'hydrogène à un débit de 5 l/min. Le lit est constitué de particules d'alumine (650g) de 300 microns de diamètre moyen. Les particules du lit sont mises en fluidisation par un mélange de méthane, d'hydrogène et d'argon. Par un ajustement du débit de ces trois gaz, on règle le temps de séjour du méthane dans le réacteur. Une bonne fluidisation est obtenue pour un débit total compris entre 15 l/min et 40 l/min. On détermine la quantité optimale d'hydrogène de fluidisation par rapport à celle du méthane de façon à minimiser la formation de noir de carbone. Ce rapport est compris entre 0,5 et 10 et de préférence entre 2 et 5. Les gaz de fluidisation sont préchauffés à une température comprise entre 50 et 500°C, de préférence entre 150°C et 350°C. Les débits de gaz plasmagènes et de fluidisation sont mesurés et régulés au moyen de débitmètres massiques. Des thermocouples sont installés pour permettre de mesurer la température des gaz de fluidisation en amont de l'enceinte, de la paroi de l'enceinte, du lit fluidisé et les températures dans le tube 5.

La température du lit fluidisé est choisie comme température de référence puisque d'une part elle caractérise l'efficacité de la trempe et d'autre part parce que la réaction de conversion a lieu dans le lit fluidisé.

L'analyse des produits se fait par chromatographie en phase gazeuse.

Les huit exemples sont détaillés dans les tableaux 1 et 2 suivants. Ils ont été effectués avec la même masse de particules identiques et avec des conditions de fonctionnement de la torche identiques. Ils diffèrent entre eux par les débits des gaz de fluidisation et par la température moyenne du lit fluidisé. Les résultats obtenus avec ces différents exemples sont également détaillés dans les tableaux 1 et 2 suivants.

## TABLEAU 1.

### CONDITIONS OPERATOIRES ET RESULTATS D'ANALYSE

| No. Ex. | Température moyenne dans le lit fluidisé °C | Gaz de fluidisation (1/min) | | | Composition molaire des produits de conversion (%) | | | |
|---|---|---|---|---|---|---|---|---|
| | | Ar | H2 | CH4 | (C2H2) | (C2H4) | (C2H5) | (C) |
| 1 | 580 | 11 | 8 | 5 | 11 | 2,5 | 0,5 | 11,5 |
| 2 | 700 | 11 | 8 | 5 | 9 | 2 | 0,4 | 17 |
| 3 | 770 | 11 | 8 | 5 | 6 | 2 | 0,4 | 30 |
| 4 | 500 | 15 | 8 | 5 | 1,5 | 0,3 | 0,1 | 5 |
| 5 | 600 | 6 | 8 | 5 | 4 | 1,5 | 0,3 | 22 |
| 6 | 600 | 0 | 8 | 5 | 7 | 2,5 | | 28 |
| 7 | 550 | 0 | 13 | 5 | 21 | 13,5 | 2 | 5 |
| 8* | 580 | 11 | 8 | 5 | 0,2 | 0 | 0 | 0,3 |

* torche arrêtée    (C)  =   concentration noir de carbone

## TABLEAU 2.

### RESULTATS

| No. Ex. | Conversion CH4 % | Sélectivité C2 % | Rendement C2 % |
|---|---|---|---|
| 1 | 34 | 71 | 24 |
| 2 | 35 | 57 | 20 |
| 3 | 43 | 40 | 17 |
| 4 | 9 | 42 | 3,8 |
| 5 | 32 | 34 | 11 |
| 6 | 43 | 38 | 16 |
| 7 | 57 | 94 | 53 |
| 8* | 0,7 | 57 | 4 |

* torche arrêtée

Les résultats obtenus et listés dans les tableaux 1 et 2 montrent tout l'intérêt du procédé et prouvent particulièrement l'efficacité de la trempe par le lit fluidisé puisque la température moyenne dans celui-ci est relativement basse (comprise entre 500°C et 800°C).

Les exemples 1 à 3 ont été effectués dans des conditions de fluidisation identiques mais à trois températures différentes. Les résultats montrent que la quantité de noir de carbone formée augmente très rapidement avec la température. Il ressort de ces résultats que le contrôle de la température est primordial et l'on comprend donc tout l'intérêt qu'il y a à tremper le plasma.

L'exemple 4 a été effectué à une température de 500°C. On remarque que le taux de conversion de $CH_4$ à cette température n'est que de 9%. En conséquence une température d'environ 500°C constitue la limite inférieure pour la conversion du méthane.

Les exemples 5 et 6 ont été effectués à une même température mais avec un débit de fluidisation différent. On constate une augmentation du taux de conversion lorsque le débit du gaz de fluidisation diminue, c'est-à-dire lorsque le temps de séjour du méthane augmente. Le contrôle de ce paramètre important peut donc se faire facilement.

Si l'on compare les exemples 6 et 7, on note une nette augmentation du rendement en C2, due certainement à l'accroissement du rapport hydrogène/méthane. Le rôle de l'hydrogène de fluidisation est multiple. Notamment il inhibe les réactions de déshydrogénation conduisant au noir de carbone et il "protège" le méthane contre les chocs thermiques, mais il peut aussi limiter la réaction de conversion, d'où la nécessité de déterminer sa concentration optimale.

L'exemple 8 a été réalisé pour observer le rôle spécifique du plasma qui est d'apporter en forte concentration des espèces radicalaires. Pour ce faire, une expérience simple a été réalisée qui consiste à arrêter la torche et à analyser aussitôt le mélange réactionnel. On remarque qu'à une même température mais sans le plasma, le taux de conversion du méthane est négligeable.

Bien que ces exemples aient été obtenus sans optimisation des paramètres opératoires, on remarquera les très bons résultats de l'exemple 7 dans lequel la sélectivité en C2 (acétylène) est de 94% c'est-à-dire déjà meilleure que celle du procédé Hüls qui est d'environ 74%.

La conversion du méthane est définie par le rapport de la quantité de méthane converti par la quantité totale de méthane introduite. Elle est calculée ainsi :

$$C = \frac{2(C2H2) + 2(C2H4) + 2(C2H6) + (C)}{2(C2H2) + 2(C2H4) + 2(C2H6) + (C) + (CH4)}$$

avec (X) concentration molaire du constituant X dans le mélange réactionnel, donnée par l'analyse chromatographique.

La sélectivité en C2 est le rapport de la quantité de produits en C2 obtenus par la quantité de produits de conversion. Elle est calculée ainsi :

$$S = \frac{2(C2H2) + 2(C2H4) + 2(C2H6)}{2(C2H2) + 2(C2H4) + 2(C2H6) + (C)}$$

Le rendement en C2 est défini par le rapport de la quantité de produits en C2 obtenue par la quantité de méthane introduite. Il est calculé ainsi

$$R = \frac{2(C2H2) + 2(C2H4) + 2(C2H6)}{2(C2H2) + 2(C2H4) + 2(C2H6) + (C) + (CH4)}$$

Bien entendu, les modes de réalisations décrits et illustrés ne sont donnés qu'à titre d'exemple. Ainsi, en fonction de la nature du catalyseur, de la charge et des conditions opératoires, il est possible d'obtenir des hydrocarbures supérieures à $C_2$. Par ailleurs, on pourrait introduire dans le lit fluidisé les produits à convertir, différemment de l'exemple représenté, c'est-à-dire séparément du gaz de fluidisation, à tout endroit approprié à condition de respecter la trempe du plasma par le lit fluidisé. Il est bien entendu également que le plasma utilisé peut être produit de façon quelconque, notamment par arc électrique soufflé ou transféré ou bien encore par induction.

## Revendications

1. Procédé de conversion de gaz naturel ou d'alcane (s) léger (s) en hydrocarbures insaturés dans lequel on met en contact un gaz naturel ou un ou des alcane(s) avec un plasma d'un gaz contenant de l'hydrogène dans un espace de réaction et on récupère lesdits hydrocarbures insaturés à une sortie dudit espace de réaction caractérisé en ce que l'on créé à l'intérieur dudit espace un lit fluidisé de particules d'un matériau réfractaire et avantageusement catalytique et en ce que l'on introduit ledit plasma et ledit gaz naturel ou le ou lesdits alcanes légers dans ledit lit de façon que celui-ci effectue la trempe du milieu réactionnel et catalyse la réaction de conversion.

2. Procédé selon la revendication 1, caractérisé en ce que ledit lit de particules est fluidisé par un courant gazeux de fluidisation contenant avantageusement de l'hydrogène.

3. Procédé selon la revendication 2, caractérisé en ce que ledit courant gazeux de fluidisation contient de l'hydrogène et de l'argon.

4. Procédé selon l'une des revendications 1, 2 ou 3, caractérisé en ce que ledit gaz naturel ou le ou lesdits alcanes légers est introduit dans le lit avec le courant gazeux de fluidisation.

5. Procédé selon l'une des revendications 1 ou 4, caractérisé en ce que l'alcane léger est du méthane.

6. Procédé selon l'une des revendications 2, 3, 4 ou 5, caractérisé en ce que l'hydrogène et le méthane sont introduits dans une proportion hydrogène/méthane allant de 0,5 à 10 et de préférence de 2 à 5.

7. Procédé selon l'une des revendications 2 à 6, caractérisé en ce que le courant gazeux de fluidisation est préchauffé à une température comprise entre 50° et 500°C et de préférence entre 150°C et 350°C.

8. Procédé selon la revendication 1, caractérisé en ce qu'il consiste à introduire un plasma contenant au moins 10% d'hydrogène.

9. Procédé selon la revendication 8, caractérisé en ce qu'il consiste à introduire un plasma contenant de l'hydrogène et de l'argon.

10. Procédé selon la revendication 1, caractérisé en ce que le lit est constitué de particules d'un matériau choisi notamment dans le groupe consistant en oxydes, carbures, nitrures et borures.

11. Procédé selon la revendication 10, caractérisé en ce que les particules possèdent un effet catalytique.

12. Procédé selon l'une des revendications 10 ou 11, caractérisé en ce que le lit contient de plus un catalyseur.

13. Procédé selon l'une des revendications précédentes, caractérisé en ce que la réaction de conversion est réalisée à une température comprise entre 500°C et 1200°C et de préférence entre 500°C et 800°C.


## Patentansprüche

1. Verfahren zur Umwandlung von Erdgas oder von einem leichten Alkan oder von leichten Alkanen in ungesättigte Kohlenwasserstoffen, bei welchem man ein Erdgas oder ein Alkan oder Alkane in Berührung mit einem Plasma eines wasserstoffhaltigen Gases in einem Reaktionsraum bringt und man die besagten ungesättigten Kohlenwasserstoffe an einem Austritt des besagten Reaktionsraumes einsammelt, dadurch gekennzeichnet, dass man innerhalb des besagten Raumes ein Wirbelschichtfliessbett aus Teilchen eines feuerfesten und vorzugsweise katalytischen Werkstoffs schafft und dass man das besagte Plasma und das besagte Erdgas oder das leichte Alkan oder die besagten leichten Alkanen in das besagte Bett einführt, so dass dieses das Abschrecken des Reaktionsmediums durchführt und die Umsatzreaktion katalysiert.

2. Verfahren nach Anspruch 1, dadurch gekennzeichnet, dass das besagte Teilchenbett durch einen vorteilhaft Wasserstoff enthaltenden gasförmigen Fluidisationsstrom fluidisiert wird.

3. Verfahren nach Anspruch 2, dadurch gekennzeichnet, dass der besagte gasförmige Fluidisationsstrom Wasserstoff und Argon enthält.

4. Verfahren nach einem der Ansprüche 1, 2 oder 3, dadurch gekennzeichnet, dass das besagte Erdgas oder das leichte Alkan oder die besagten leichten Alkanen in das Bett mit dem gasförmigen Fluidisationstrom eingeführt wird.

5. Verfahren nach einem der Ansprüche 1 oder 4, dadurch gekennzeichnet, dass das leichte Alkan Methan ist.

6. Verfahren nach einem der Ansprüche 2, 3, 4 oder 5, dadurch gekennzeichnet, dass der Wasserstoff und das Methan in einem Wasserstoff/Methan-Verhältnis von 0,5 bis 10 und vorzugsweise von 2 bis 5 eingeführt werden.

7. Verfahren nach einem der Ansprüche 2 bis 6, dadurch gekennzeichnet, dass der gasförmige Fluidisationsstrom bis zur einer zwischen 50° und 500°C und vorzugsweise zwischen 150°C und 350°C liegenden Temperatur vorerwärmt wird.

8. Verfahren nach Anspruch 1, dadurch gekennzeichnet, dass es darin besteht, ein wenigstens 10% Wasserstoff enthaltendes Plasma einzuführen.

9. Verfahren nach Anspruch 8, dadurch gekennzeichnet, dass es darin besteht, ein Wasserstoff und Argon enthaltendes Plasma einzuführen.

10. Verfahren nach Anspruch 1, dadurch gekennzeichnet, dass das Bett aus Teilchen eines insbesondere in der aus Oxiden, Karbiden, Nitriden und Boriden bestehenden Gruppe gewählten Werkstoffs gebildet wird.

11. Verfahren nach Anspruch 10, dadurch gekennzeichnet, dass die Teilchen eine katalytische Wirkung besitzen.

12. Verfahren nach einem der Ansprüche 10 oder 11, dadurch gekennzeichnet, dass das Bett ausserdem einen Katalysator enthält.

13. Verfahren nach einem der vorangehenden Ansprüche, dadurch gekennzeichnet, dass die Umwandlungsreaktion bei einer zwischen 150°C und 1200°C und vorzugsweise zwischen 500°C und 800°C liegenden Temperatur durchgeführt wird.

## Claims

1. Method of conversion of natural gas or of light alkane(s) into unsaturated hydrocarbons, wherein one puts a natural gas or one light alkane or light akanes in contact with a plasma of a gas containing hydrogen in a reaction space and one recovers the said unsaturated hydrocarbons at one outlet of the said reaction space, characterized in that one creates inside of the said space a fluidized bed of particles of a refractory and advantageously catalytic material and in that one introduces the said plasma and the said natural gas or the light alkane or the said light alkanes into the said bed so that the latter effects the quenching of the reactive medium and catalyses the conversion reaction.

2. Method according to claim 1, characterized in that the said bed of particles is fluidized by a gaseous fluidization stream advantageously containing hydrogen.

3. Method according to claim 2, characterized in that the said gaseous fluidization stream contains hydrogen and argon.

4. Method according to one of claims 1, 2 or 3, characterized in that the said natural gas or the light alkane or the said light alkanes is introduced into the bed with the gaseous fluidization stream.

5. Method according to one of claims 1 or 4, characterized in that the light alkane is methane.

6. Method according to one of claims 2, 3, 4 or 5 characterized in that the hydrogen and the methane are introduced in a hydrogen/methane proportion ranging from 0.5 to 10 and preferably from 2 to 5.

7. Method according to one of claims 2 to 6, characterized in that the gaseous fluidization stream is preheated to a temperature lying between 50° and 500°C and preferably between 150°C and 350°C.

8. Method according to claim 1, characterized in that it consists in introducing a plasma containing at least 10% of hydrogen.

9. Method according to claim 8, characterized in that it consists in introducing a plasma containing hydrogen and argon.

10. Method according to claim 1, characterized in that the bed consists of particles of a material selected in particular from the group consisting of oxides, carbides, nitrides or borides.

11. Method according to claim 10, characterized in that the particles have a catalytic effect.

12. Method according to one of claims 10 or 11, characterized in that the bed in addition contains a catalyst.

13. Method according to one of the foregoing claims, characterized in that the conversion reaction is carried out at a temperature comprised between 500°C and 1,200°C and preferably between 500°C and 800C.